# EUROPÄISCHE PATENTANMELDUNG

(11) **EP 3 881 796 A1**
(43) Veröffentlichungstag der Anmeldung: **22.09.2021**
(21) Anmeldenummer: 20163574.5
(22) Anmeldetag: 17.03.2020
(51) Int. Cl.: A61C 9/00, A61B 1/24

(54) **VERFAHREN ZUM OPTISCHEN ERFASSEN DER DREIDIMENSIONALEN OBERFLÄCHENGEOMETRIE VON INTRAORALEN STRUKTUREN**

(71) Anmelder: a.tron3d GmbH, 9020 Klagenfurt am Wörthersee (AT)
(72) Erfinder: JESENKO, Juergen, 9587 Riegersdorf (AT); VON SEE, Constantin, 3500 Krems/Imbach (AT)
(74) Vertreter: Beer & Partner Patentanwälte KG

(57) **Zusammenfassung**

Bei einem Verfahren zum optischen Erfassen der dreidimensionalen Oberflächengeometrie von intraoralen Strukturen, bei dem die intraoralen Strukturen Zähne, hartes Gewebe und/oder künstliche Strukturen, die im Wesentlichen entlang eines Kieferbogens verlaufen, sowie weiches Gewebe beinhalten, wird eine Schablone mit einem Abdeckrahmen im Bereich der intraoralen Strukturen angeordnet, und das weiche Gewebe im Bereich der Schablone während des Erfassens im Wesentlichen durch den Abdeckrahmen der Schablone wird abgedeckt.

## Beschreibung

Die Erfindung betrifft ein Verfahren zum optischen Erfassen der dreidimensionalen Oberflächengeometrie von intraoralen Strukturen, wobei die intraoralen Strukturen Zähne, hartes Gewebe und/oder künstliche Strukturen, die im Wesentlichen entlang eines Kieferbogens verlaufen, sowie weiches Gewebe beinhalten.

In der digitalen Dentaltechnik sind verschiedene Methoden zum Erfassen der Zähne bereits umfangreich beschrieben. Es hat sich allerdings gezeigt, dass intraorale Oberflächen, die nicht Teil der Zähne sind, wie beispielsweise verschiedene Gewebe, häufig nicht hinreichend präzise erfasst werden oder, wenn sie erfasst werden, die Aufnahmen in ihrer Gesamtheit sogar verschlechtern.

Insbesondere weiche Gewebe, die sehr leicht ihre relative Lage zu den Zähnen ändern, sind dabei eine große Fehlerquelle.

Die bisherigen Bemühungen, diese Fehlerquelle zu vermeiden oder wenigstens zu vermindern, waren vor allem auf Seiten der Datenverarbeitung des erfassten Modells, d.h. der erfassten Oberflächengeometrie, angesiedelt. Ein Beispiel für das rechnerische Entfernen von weichem Gewebe aus dem Modell zeigt beispielsweise die EP 3 308 738 A, bei dem links und rechts des Kiefers Rauminformationen, die eine bestimmte Entfernung vom Kiefer überschreiten, aus dem Modell entfernt werden. Andere rechnerische Lösungen verwenden beispielsweise Farbinformationen oder ermitteln, welche Oberflächen sich während des Aufnehmens stärker verändern als andere.

Allen bekannten Methoden ist gemeinsam, dass sie zum einen Rechenzeit und -ressourcen benötigen, die häufig mit hohen Kosten für leistungsstärkere Systeme verbunden sind, und dass sie zum anderen fehleranfällig.

Der Erfindung liegt daher die Aufgabe zu Grunde, die oben beschrieben Nachteile zu überwinden.

Gelöst wird diese Aufgabe erfindungsgemäß durch ein Verfahren mit den Merkmalen des Anspruchs 1.

Es ist wichtig, im Rahmen der Beschreibung der Erfindung begrifflich zwischen hartem und weichem Gewebe zu unterscheiden. Stark vereinfacht kann man sagen, dass, von der Mukogingivallinie aus betrachtet, die zahnseitigen Gewebe harte Gewebe, und die übrigen, auf der anderen Seite der Mukogingivallinie liegenden Gewebe weiche Gewebe sind.

Neben Zähnen und hartem Gewebe können dem Zahnbogen im Rahmen der Erfindung auch künstliche Strukturen folgen. Bei diesen kann es sich beispielsweise um Zahnersatz handeln, der die Stelle der Zähne eingenommen hat und nicht immer mit optischen Mitteln von Zähnen unterschieden werden kann. Eine weitere Möglichkeit für derartige künstliche Strukturen können beispielsweise auch Zahnspangen und andere Mittel zur Regulierung der Zahnstellung sein. Zahnersatz, der sich zwar anstelle der Zähne im Zahnbogen befindet, aber deutlich von solchen zu unterscheiden ist, ist ebenfalls möglich. Dabei kann es sich beispielsweise um ein Heilabutment handeln.

Beim erfindungsgemäßen Verfahren zum optischen Erfassen der dreidimensionalen Oberflächengeometrie von intraoralen Strukturen, bei dem die intraoralen Strukturen Zähne, hartes Gewebe und/oder künstliche Strukturen, die im Wesentlichen entlang eines Kieferbogens verlaufen, sowie weiches Gewebe beinhalten, wird eine Schablone mit einem Abdeckrahmen im Bereich der intraoralen Strukturen angeordnet, und das weiche Gewebe im Bereich der Schablone wird während des Erfassens im Wesentlichen durch den Abdeckrahmen der Schablone abgedeckt.

Da das weiche Gewebe im Bereich der Schablone während des Erfassens der dreidimensionalen Oberflächengeometrie im Wesentlichen durch den Abdeckrahmen der Schablone abgedeckt wird, hat es während des Erfassens keinen störenden Einfluss, sodass die erfasste Geometrie weniger Fehler aufweist bzw. ein nachträgliches Bereinigen des Modells vermieden oder wenigstens vereinfacht werden kann.

Gemäß einer bevorzugten Weiterbildung des erfindungsgemäßen Verfahrens weist der Abdeckrahmen der Schablone eine mit dem Kieferbogen oder einem Bereich des Kieferbogens korrespondierende Ausnehmung auf, in der der Kieferbogen während des Erfassens aufgenommen ist. Durch diese Ausnehmung können alle intraoralen Strukturen aufgenommen werden, die erfasst werden sollen, während das weiche Gewebe verdeckt ist und die Messungen nicht negativ beeinflussen kann.

Bei alternativen Ausführungsformen ohne Ausnehmung kann man beispielsweise einen bogenförmigen Abdeckrahmen nur auf einer Seite des Kieferbogens auf das weiche Gewebe (beispielsweise zwischen Kieferbogen und Wangen) legen.

Gemäß einer weiteren bevorzugten Ausführungsform des Verfahrens werden während des Erfassens Markierungen auf der Schablone erkannt. Diese können, abhängig von der gewählten Art der Markierung, verschiedene Zwecke erfüllen. Bekannte Abstände und Maße der Markierungen können beispielsweise die Genauigkeit des Erfassens erhöhen.

Gemäß noch einer weiteren bevorzugten Ausführungsform des erfindungsgemäßen Verfahrens wird während des Erfassens eine Farbe des Abdeckrahmens der Schablone erkannt. Dadurch kann (bei entsprechender Farbwahl) die Unterscheidung zwischen abgedeckter und nicht zu erfassender Oberfläche und nicht abgedeckter und zu erfassender Oberfläche besonders leicht getroffen werden.

Selbstverständlich ist auch eine Kombination denkbar, bei der Markierungen, die gegebenenfalls auch mehrere verschiedene Farben haben können, erkannt werden. Markierungen in vorkalibrierten Farben können beispielsweise zur Verbesserung der Farbtreue verwendet werden.

Gemäß einer Weiterbildung der Erfindung kann in einem computerimplementierten Verfahren zunächst eine Schablone erzeugt werden, die dann zum Durchführen des erfindungsgemäßen Verfahrens verwendet wird. Die Schablone kann beispielsweise mithilfe eines 3D-Druckers hergestellt werden. Hierfür können beispielsweise Daten der intraoralen Strukturen hinzugezogen werden, die bereits im Vorfeld gewonnen wurden. Es können aber auch übliche Kieferformen hinzugezogen werden, die dem statistischen Mittel üblicher Kieferformen entsprechen.

Weitere bevorzugte Ausführungsformen der Erfindung sind Gegenstand der übrigen Unteransprüche.

Nachstehend ist ein bevorzugtes Ausführungsbeispiel der Erfindung anhand der Zeichnungen näher beschrieben. Es zeigt:
- Fig. 1: eine Schablone zum Ausführen des erfindungsgemäßen Verfahrens,
- Fig. 2: eine Ansicht der Schablone von Fig. 1 in Verwendung, und
- Fig. 3: eine alternative Ausführungsform einer Schablone.

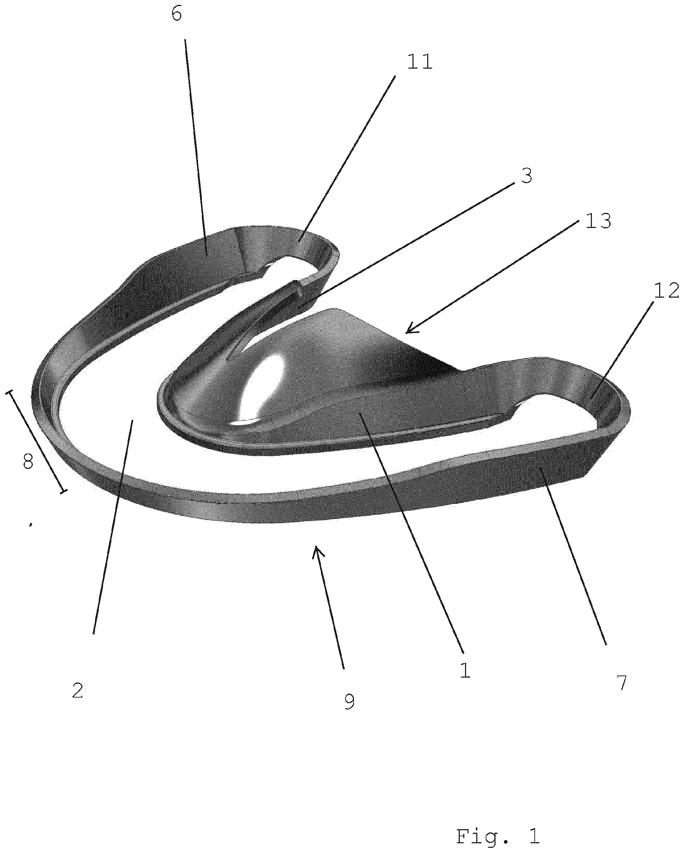

Fig. 1 zeigt eine mögliche Ausführungsform einer Schablone 1 mit einer Ausnehmung 2 zum Ausführen des erfindungsgemäßen Verfahrens. Die in Fig. 1 gezeigte Schablone 1 ist insbesondere zum Erfassen des Kieferbogens des Unterkiefers geeignet. Eine vergleichbar gestaltete Schablone kann aber natürlich auch für den Oberkiefer verwendet werden. Die genaue Form bzw. die genauen Abmessungen der Schablone 1 sind für die Ausführbarkeit des Verfahrens nebensächlich, sofern die folgenden, grundlegenden Bedingungen erfüllt sind:
- Die Schablone muss klein genug sein, um sich in einem Mundraum platzieren zu lassen.
- Die Schablone muss eine Ausnehmung 2 oder einen Rand aufweisen, die bzw. der wenigstens bereichsweise dem Verlauf eines Kieferbogens folgt.

Darüber hinaus kann die Schablone bevorzugt, aber nicht zwingend, beispielsweise eine oder mehrere der folgenden Eigenschaften aufweisen:
- Sie ist aus einem für einen optischen Scanner leicht von intraoralen Strukturen unterscheidbaren Material oder mit einer entsprechenden Oberfläche hergestellt.
- Sie weist Muster auf.
- Sie hat eine besonders stark zum Mundinnenraum kontrastierende Farbe, beispielsweise Grün.
- Sie ist aus einem flexiblen Material gefertigt.
- Wenn sie speziell für das Durchführen des Verfahrens am Unterkiefer gedacht ist, kann die Schablone (wie in Fig. 1 dargestellt) einen Spalt 3 aufweisen, der mit dem Zungenbändchen korrespondiert und für dieses Raum gibt.
- Sie kann als Set in wenigstens zwei, vorzugsweise mehr, verschiedenen Größen und/oder Formen zur Verfügung gestellt werden, um beispielsweise für Kinder und Erwachsene jeweils geeignete Schablonen zur Verfügung zu stellen, wobei vor dem Erfassen der dreidimensionalen Oberflächengeometrie eine geeignete Größe bzw. Form aus dem Set ausgewählt wird.
- Sie kann aus einem thermoplastischen Material hergestellt sein. So kann sie schnell und einfach an die jeweiligen intraoralen Gegebenheiten angepasst werden.

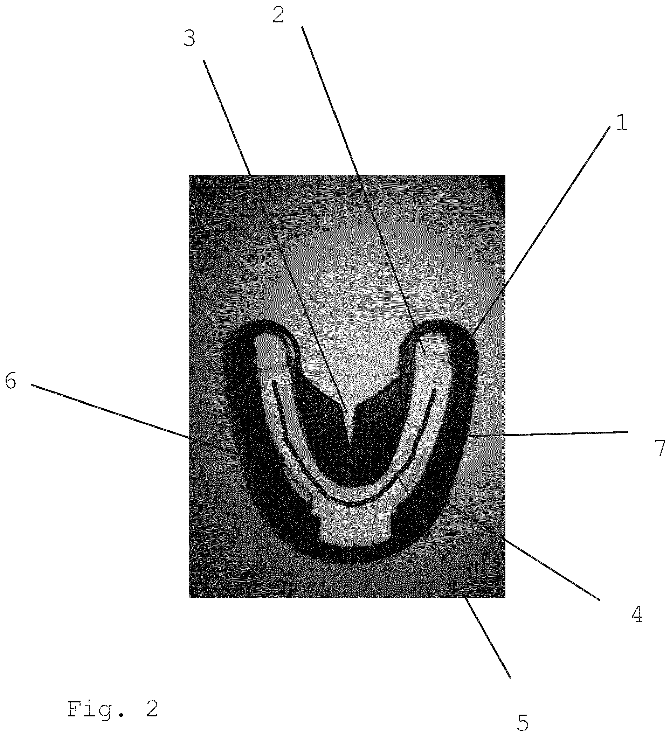

Fig. 2 zeigt die Schablone von Fig. 1 in einer Verwendungslage an einem Kieferbogen 4, dessen Verlauf symbolisch durch eine Linie 5 gezeigt ist. Man kann erkennen, dass die Ausnehmung 2 in der Schablone 1 dem Verlauf 5 des Kieferbogens 4 im Wesentlichen folgt.

Vom Kieferbogen 4 bzw. der Ausnehmung 2 aus betrachtet außen liegende Abschnitte der Schablone können zu Flügeln 6, 7 ausgeformt sein. Die Flügel 6, 7, die den wirksamen Bereich der Schablone verbreitern, können den Scanvorgang erleichtern, indem sie die Wangeninnenseiten leicht nach außen drücken und so eine bessere Übersicht ermöglichen. Um diesen Vorgang für einen Patienten, dessen Mundraum gescannt werden soll, angenehmer zu gestalten, kann das Material der Schablone flexibel gestaltet sein.

Die Flügel 6, 7 treffen sich in einem vorderen Mittelbereich 8 und bilden gemeinsam mit diesem einen Außenteil 9 der Schablone. Der Außenteil 9 ist über, vorzugsweise flexible, Stege 11, 12 mit einem Innenteil 13 verbunden. Ausführungsformen, die keine Ausnehmung 2 aufweisen, können beispielsweise ohne den Innenteil 13 gefertigt sein. In diesem Fall begrenzt der kieferseitig gelegene Rand anstelle der Ausnehmung den Übergang zwischen weichem und hartem Gewebe. Die Stege 11, 12 können bei einer solchen Ausführungsform wegfallen oder dennoch vorhanden sein.

Soll die Schablone 1 im Bereich des Unterkiefers angewendet werden, ist es vorteilhaft, einen Spalt 3 im Innenteil 13 anzuordnen, der Raum für das Zungenbändchen gibt.

Ein beispielhaftes, erfindungsgemäßes Verfahren könnte wie folgt durchgeführt werden:
- Zuerst wird die Schablone in dem Bereich, der erfasst werden soll, positioniert. Durch das Positionieren der Schablone wird das weiche Gewebe in der Umgebung der Objekte, die erfasst werden sollen, wenigstens bereichsweise abgedeckt.
- Der Bereich wird inklusive Schablone oder wenigstens Teilen dieser erfasst.
- Optional kann die Schablone entweder schon während des Erfassens oder in einer darauffolgenden Interpretation der erfassten Daten erkannt und gegebenenfalls aus dem erfassten 3D-Datensatz entfernt werden.
- Wird der vorgenannte Schritt nicht ausgeführt, kann alternativ auch einfach mit der erfassten Oberfläche, die sich aus der erfassten Oberfläche der intraoralen Strukturen und der erfassten Oberfläche der Schablone zusammensetzt, gearbeitet werden. Da die Schablone erfindungsgemäß weiches Gewebe wenigstens bereichsweise abdeckt, werden die negativen Effekte, die sich durch das Erfassen von weichem Gewebe ergeben, automatisch minimiert, und auch in dieser sehr einfachen Ausführungsform der Erfindung stellt sich bereits eine Verbesserung ein.

Komplexere Ausführungsformen können beispielsweise eine - gegebenenfalls bekannte - Farbe der Schablone berücksichtigen, um diese vergleichbar mit einem inversen Green-Screen rechnerisch aus der Aufnahme zu entfernen oder mittels der Schablone einen Farbabgleich durchzuführen, um eine Farbe des Gewebes und der Zähne möglichst realitätsnahe zu erfassen. Wurde die Farbe erfasst, kann so später ein möglichst natürlich wirkender Zahnersatz geschaffen werden.

Eine weitere Möglichkeit eines komplexeren Verfahrens beinhaltet beispielsweise, dass die Schablone geometrische Markierungen aufweist und diese im Rahmen des Verfahrens genutzt werden, um Oberflächen mit einer höheren räumlichen Präzision zu erfassen.

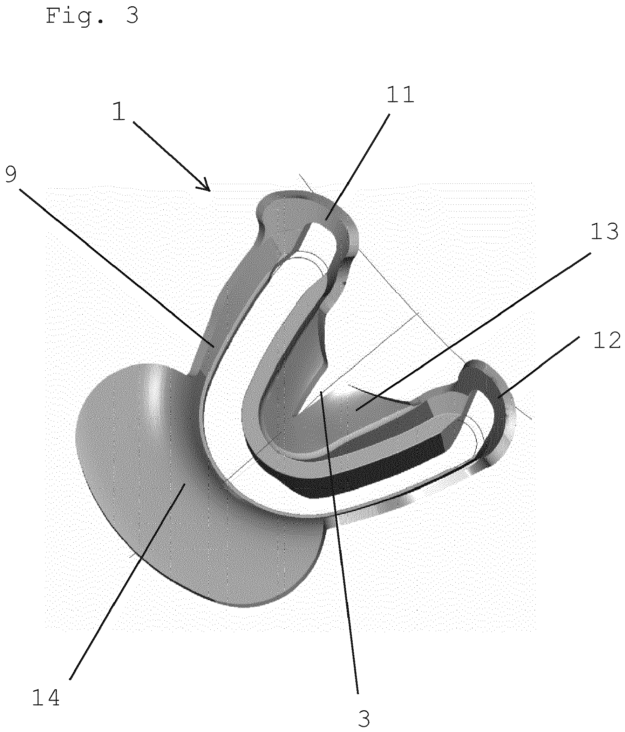

Die in Fig. 3 gezeigte Ausführungsform unterscheidet sich von der in den Fig. 1 und 2 gezeigten Ausführungsform durch einen Griffbereich 14. Gemäß einer bevorzugten Durchführungsform des erfindungsgemäßen Verfahrens kann die Schablone während des Erfassens durch die Bedienperson am Griffbereich in Position gehalten werden. Dadurch ist es möglich, eventuellen Muskelbewegungen der Kiefermuskeln entgegenzuwirken, die die Schablone verschieben und damit das Erfassen negativ beeinträchtigen könnten.

### Bezugszeichenliste:

- 1: Schablone
- 2: Ausnehmung
- 3: Spalt
- 4: Kieferbogen
- 5: Linie (Verlauf)
- 6: Flügel
- 7: Flügel
- 8: vorderer Mittelbereich
- 9: Außenteil / bukkaler Abschnitt
- 10: -nicht vergeben-
- 11: Steg
- 12: Steg
- 13: Innenteil / lingualer Abschnitt
- 14: Griffbereich

## Patentansprüche

1. Verfahren zum optischen Erfassen der dreidimensionalen Oberflächengeometrie von intraoralen Strukturen, bei dem die intraoralen Strukturen Zähne, hartes Gewebe und/oder künstliche Strukturen, die im Wesentlichen entlang eines Kieferbogens verlaufen, sowie weiches Gewebe beinhalten, **dadurch gekennzeichnet, dass** eine Schablone mit einem Abdeckrahmen im Bereich der intraoralen Strukturen angeordnet wird, und dass das weiche Gewebe im Bereich der Schablone während des Erfassens im Wesentlichen durch den Abdeckrahmen der Schablone abgedeckt wird.

2. Verfahren nach Anspruch 1, **dadurch gekennzeichnet, dass** der Abdeckrahmen der Schablone eine mit dem Kieferbogen oder einem Bereich des Kieferbogens korrespondierende Ausnehmung aufweist, in der der Kieferbogen während des Erfassens aufgenommen ist.

3. Verfahren nach Anspruch 1 oder 2, **dadurch gekennzeichnet, dass** während des Erfassens oder danach Markierungen auf der Schablone erkannt werden.

4. Verfahren nach einem der Ansprüche 1 bis 3, **dadurch gekennzeichnet, dass** während des Erfassens oder danach eine Farbe des Materials der Schablone erkannt wird.

5. Verfahren nach einem der Ansprüche 1 bis 4, **dadurch gekennzeichnet, dass** eine Schablone aus einem flexiblen Material verwendet wird.

6. Verfahren nach einem der Ansprüche 1 bis 5, **dadurch gekennzeichnet, dass** ein Set von Schablonen in verschiedenen Größen zur Verfügung gehalten wird und vor dem Erfassen eine Auswahl einer geeigneten Größe erfolgt.

7. Verfahren nach einem der Ansprüche 1 bis 6, **dadurch gekennzeichnet, dass** der Abdeckrahmen aus einem Thermoplast besteht und vor dem Erfassen an den Mundinnenraum bzw. den Kieferbogen angepasst wird.

8. Verfahren nach einem der Ansprüche 1 bis 7, **dadurch gekennzeichnet, dass** der Abdeckrahmen einen Griffbereich aufweist und während des Erfassens durch eine Bedienperson am Griffbereich in Position gehalten wird.

9. Computerimplementiertes Verfahren zum Erzeugen einer Schablone, **dadurch gekennzeichnet, dass** die Schablone, nachdem sie erzeugt wurde, zum Ausführen eines Verfahrens nach einem der Ansprüche 1 bis 8 verwendet wird.
